# EUROPEAN PATENT APPLICATION

(11) **EP 0 834 740 A1**
(43) Date of publication of application: **08.04.1998**
(21) Application number: 97915706.2
(22) Date of filing: 10.04.1997
(51) Int. Cl.: G01N 33/53

(54) **ANTI-GLU17-OSTEOCALCIN ANTIBODY**

(30) Priority: 10.04.1996 JP 88608/96; 27.02.1997 JP 43331/97
(71) Applicant: Eisai Co., Ltd., Tokyo 112-88 (JP)
(72) Inventor: SAKAKIBARA, Shunpei, Osaka 565 (JP); KIMURA, Terutoshi, Hyogo 662 (JP); MORIMOTO, Shigeto Imperial Shato 701, Osaka 547 (JP)
(74) Representative: Hansen, Bernd, Dr. Dipl.-Chem.
(86) International application number: JP9701246
(87) International publication number: WO9738309

(57) **Abstract**

Provided is an antibody capable of discriminating Glu¹⁷-osteocalcin from an osteocalcin.

More specifically, provided is an anti-Glu¹⁷-osteocalcin antibody or fragment thereof which specifically binds to Glu¹⁷-osteocalcin which is an osteocalcin having a Glu residue at position 17 or osteocalcin fragment containing the 17th Glu residue.

## Description

### Technical Field

The present invention relates to an anti-Glu¹⁷-osteocalcin antibody which is useful as a reagent for detecting or measuring a low-carboxylated osteocalcin, particularly Glu¹⁷-osteocalcin (which means an osteocalcin having a glutamic acid [Glu] residue at the 17th position from the N-terminal; this definition will be applied equally hereinafter); and a measuring method of Glu¹⁷-osteocalcin in a sample by using the antibody.

### Prior Art

### 〈Background of the Invention〉

As factors taking part in a decrease in a bone mineral density which tends to occur in women at the involutional period, pointed out have been a postmenopausal reduction of female sex hormone in the blood, change in the metabolism of vitamin D, deficiency of calcium (Ca) intake, lack of exercise and smoking [Aloia J. Cohn S, Vaswani AN, Yeh JK, Yen K, Ellis K. 1985, "Risk factors for postmenopausal osteoporosis", Am. J. Med. 78, 95-100(1985)"]. In recent days, it has been reported that female patients suffering from osteoporosis, followed by bone fracture show a decrease in the blood levels of vitamins K₁ and K₂ [Hart JP, Shearer MJ, Klenerman L, Catterall A, Reeve J. Sambrook PN, Dodds RA, Bitensky L, Chayen J., "Electrochemical detection of depressed circulating levels of vitamin K₁ in osteoporosis", J. Clin. Endocrinol. Metab., 60, 1268-1269(1985); Hodges SJ, Akesson K, Vergnaud P, Obrant K, Delmas PD., "Circulating levels of vitamins K₁ and K₂ decreased in elderly women with hip fracture", J. Bone Miner. Res., 10, 1241-1245(1993)]. It has also been reported that the administration of MK-4, which is a kind of vitamin K₂, brought about an increase in a bone mineral density of women at the involutional period [Orimo H, Fujita T, Onomura T, Inoue T, Kushida K, Shiraki M., "Clinical evaluation of Ea-0167 (menatetrenone) in the treatment of osteoporosis. Phase III double-blind multi-center comparative study with alfacalcidol", Clin. Eval., 20, 45-100(1992)]. Thus, it has been elucidated that the metabolism of vitamin K also has an influence on the decrease in a bone mineral density of women at the involutional period.

There have been various reports on the site of action of vitamin K on the bone metabolism. Among them, it is known that vitamin K is essential for the γ-carboxylation of a Glu residue in an osteocalcin which is a main protein among non-collagenous proteins of a bone substrate [Price PA, Fraser JD, Metz VG, "Molecular coning of matrix Gla protein: Implications for substrate recognition by the vitamin K-dependent gamma-carboxylase", Proc. Natl. Acad. Sci. USA, 84, 8335-8339(1987)] and that a γ-carboxylglutamic acid residue (Gla residue) has high affinity with Ca and only by the γ-carboxylation of the Glu residue, the osteocalcin exhibits binding ability with hydroxyapatite, which is a bone component [Hauschka PV, Lian JB, Cole DE, et al., "Osteocalcin and matrix Gla protein: vitamin K-dependent protein in bone", Physiol. Rev., 69, 990-1047(1989)]. In the cases of fracture of elderly women, particularly those suffering a fracture, an increase in the amount of low-carboxylated (or non-carboxylated) osteocalcin [in which at least one Gla residue has been replaced by a like number of a Glu residue has been reported [Plantalech L, Guillaumont M, Vergnaud P, Leclercq M, Delmas PD., "Impairment of gamma carboxylation of circulating osteocalcin (bone Gla protein) in elderly women", J. Bone Miner. Res., 6, 1211-1216(1991)], which suggests the influence, on a bone mineral density decrease in the involutional period, of an increase of low-carboxylated osteocalcin caused by insufficiency in the action of vitamin K.

The osteocalcin is also called "Bone Gla Protein (BGP)" or "vitamin K-dependent calcium binding protein". It is a protein biosynthesized by an osteoblast and is composed of 49 to 50 amino acids (said osteocalcin having a molecular weight of about 6,000 and being composed of 49 amino acids in the case of human or bovine osteocalcin, while 50 amino acids in the case of rat's osteocalcin). It is said to participate in the maintenance of homeostasis of Ca in the living organism.

Human osteocalcin has an amino acid sequence indicated by Sequence ID No. 4 (in the sequence, Xaa represents a Gla residue. This definition will be applied equally in the case where the sequence number is listed) and contains three Gla residues at positions 17, 21 and 24 from the N-terminal. The existence of three Gla residues permits the existence of 7 (2³-1=7) variant types of low-carboxylated osteocalcin.

### 〈Prior Art〉

As described above, if the circulating level of the low carboxylated (or non-carboxylated) osteocalcin can be measured as a marker of bone metabolism, it is useful for judging the risk of fracture due to osteofragility or osteoporosis.

For measurement, adopted is, for example, a method in which the content of the low-carboxylated osteocalcin is measured after the normal (carboxylated) osteocalcin is caused to adsorb to hydroxyapatite by making use of the affinity of the former with the latter [Plantalech L, Guillaumont M, Vergnaud P, Leclercq M, Delmas PD, "Impairment of gamma carboxylation of circulating osteocalcin (bone Gla protein) in elderly women, J. Bone Miner Res., 6, 1211-1216(1991)].

Delmas et al. have disclosed in JP-A-6-78788 an examining method of the risk of osteofragility or osteoporosis accompanied with fracture by measuring the concentration of the low-carboxylated osteocalcin in a biological sample. In the example of it, the concentration of the osteocalcin which has remained after adsorption to hydroxyapatite is measured using an antibody.

Those conventional methods, however, are only for the indirect measurement of the osteocalcin which has not been adsorbed to hydroxyapatite but not for the direct measurement of the low-carboxylated osteocalcin.

There has been a report on a monoclonal antibody specific to the low-carboxylated osteocalcin and also a measuring system using the monoclonal antibody, as disclosed in JP-A-6-78788. It however does not describe which was measured, a non-carboxylated osteocalcin at position 17, 21 or 24 so that it has not been elucidated which non-carboxylated osteocalcin takes an important role in the morbid condition of various bone diseases or physiological bone metabolism.

With a view to overcoming the above-described problems, the present inventors have carried out an extensive investigation on an anti-low-carboxylated osteocalcin antibody. As a result, they revealed that non-carboxylation of a γ-carboxylated glutamic acid residue at position 17 occurs more easily than that at another position under physiological conditions and also showed that among seven variant types of the low-carboxylated osteocalcin, osteocalcin having a Glu residue at position 17 is important as an essential state of low-carboxylated osteocalcin [Nakao M, Nishiguchi Y, Nakata M, Kimura T, Sakakibara S. "Synthesis of human osteocalcins: g-carboxyglutamic acid at position 17 is essential for a calcium-dependent conformational transition", Peptide Res., 7, 171-174(1994)]. Under such findings, it has also been found that anti-Glu¹⁷-osteocalcin antibody obtained using a peptide fragment indicated by Sequence ID No. 5 as an antigen has an excellent selective binding property with Glu¹⁷-osteocalcin and Gla¹⁷-osteocalcin, leading to the completion of the invention.

### SUMMARY OF THE INVENTION

The present invention therefore provides 1) an anti-Glu¹⁷-osteocalcin antibody or fragment thereof which specifically binds to Glu¹⁷-osteocalcin which is an osteocalcin having a Glu residue at position 17 thereof or to an osteocalcin fragment containing the 17th Glu residue; 2) an anti-Glu¹⁷-osteocalcin antibody or fragment thereof as described above in 1) which specifically binds to Gla²¹-osteocalcin which is an osteocalcin having a Gla residue at position 21 or to an osteocalcin fragment containing the 21st Gla residue; 3) an anti-Glu¹⁷-osteocalcin antibody or fragment thereof as described above in 1) or 2) which specifically binds to a peptide fragment indicated by Sequence ID No. 1 or 2, more specifically, an anti-Glu¹⁷-osteocalcin antibody or fragment thereof as described above in 1) or 2) which specifically binds to a peptide fragment of Tyr Leu Tyr Gln Trp Leu Gly Ala Pro Val Pro Tyr Pro Asp Pro Leu Glu Pro Arg Arg Gla Val Cys Gla/Glu Leu Asn Pro Asp Cys Asp Glu Leu Ala Asp His Ile Gly Phe Gln Glu Ala Tyr Arg Arg Phe Tyr Gly Pro Val (Sequence ID No. 1 or 2); 4) an anti-Glu¹⁷-osteocalcin antibody or fragment thereof as described above in 1) to 3) which does not bind to a peptide fragment indicated by Sequence No. 3 or 4, more specifically, an anti-Glu¹⁷-osteocalcin antibody or fragment thereof as described above in 1) to 3) which does not bind to a peptide fragment of Tyr Leu Tyr Gln Trp Leu Gly Ala Pro Val Pro Tyr Pro Asp Pro Leu Gla Pro Arg Arg Gla Val Cys Gla/Glu Leu Asn Pro Asp Cys Asp Glu Leu Ala Asp His Ile Gly Phe Gln Glu Ala Tyr Arg Arg Phe Tyr Gly Pro Val (Sequence ID No. 3 or 4); 5) an anti-Glu¹⁷-osteocalcin antibody or fragment thereof as described above in 1) to 4) which specifically binds to a peptide fragment indicated by Sequence ID No. 5 or fragment thereof containing a Glu residue, more specifically, an anti-Glu¹⁷-osteocalcin antibody or fragment thereof as described above in 1) to 4) which specifically binds to a peptide fragment of Val Pro Tyr Pro Asp Pro Leu Glu Pro Arg Arg Gla Val (Sequence ID No. 5) or fragment thereof containing a Glu residue; 6) an anti-Glu¹⁷-osteocalcin antibody or fragment thereof as described above in 1) to 5), which binds to a peptide fragment indicated by Sequence ID No. 5 or fragment thereof containing a Glu residue and does not bind to a peptide fragment indicated by Sequence ID No. 6, more specifically, an anti-Glu¹⁷-osteocalcin antibody or fragment thereof as described above in 1) to 5), which binds to a peptide fragment of Val Pro Tyr Pro Asp Pro Leu Glu Pro Arg Arg Gla Val (Sequence ID No. 5) or fragment thereof containing a Glu residue and does not bind to a peptide fragment of Val Pro Tyr Pro Asp Pro Leu Gla Pro Arg Arg Gla Val (Sequence ID No. 6); 7) an anti-Glu¹⁷-osteocalcin antibody or fragment thereof as described above in 1) to 6) which has been obtained using as an antigen a peptide fragment indicated by Sequence ID No. 5 or fragment thereof containing a Glu residue, more specifically, an anti-Glu¹⁷-osteocalcin antibody or fragment thereof as described above in 1) to 6) which has been obtained using as an antigen a peptide fragment of Val Pro Tyr Pro Asp Pro Leu Glu Pro Arg Arg Gla Val (Sequence ID No. 5) or fragment thereof containing a Glu residue; 8) an anti-Glu¹⁷-osteocalcin antibody or fragment thereof as described above in 1) to 7), wherein the antibody is a monoclonal antibody; 9) a reagent for detecting Glu¹⁷-osteocalcin, which comprises an anti-Glu¹⁷-osteocalcin antibody or fragment thereof as described above in 1) to 8); and 10) a method for measuring Glu¹⁷-osteocalcin in a sample, which comprises labeling Glu¹⁷-osteocalcin with a fluorescent or light-emitting substance, an enzyme or a radioisotope and detecting the Glu¹⁷-osteocalcin in accordance with the competitive assay by using an anti-Glu¹⁷-osteocalcin antibody or fragment thereof as described above in 1) to 8).

### DETAILED DESCRIPTION OF THE INVENTION

The present invention will hereinafter be described more specifically.

The present invention provides an antibody which selectively binds to Glu¹⁷-osteocalcin but does not bind to Gla¹⁷-osteocalcin.

The antibody according to the present invention specifically binds to Glu¹⁷-osteocalcin which is an osteocalcin having a Glu residue at position 17 from the N terminal or to an osteocalcin fragment which contains the 17th Glu residue.

The osteocalcin fragment containing the 17th Glu residue is preferred to be a peptide fragment which contains the Glu residue at position 17 and contains at least 6 amino acid residues in total, with a peptide fragment containing a Gla residue at position 21 being more preferred. In addition, it is still more preferred that it has a bonding property with a peptide fragment of Tyr Leu Tyr Gln Trp Leu Gly Ala Pro Val Pro Tyr Pro Asp Pro Leu Glu Pro Arg Arg Gla Val Cys Gla/Glu Leu Asn Pro Asp Cys Asp Glu Leu Ala Asp His Ile Gly Phe Gln Glu Ala Tyr Arg Arg Phe Tyr Gly Pro Val (Sequence ID No. 1 or 2) and does not bind to a peptide fragment of Tyr Leu Tyr Gln Trp Leu Gly Ala Pro Val Pro Tyr Pro Asp Pro Leu Gla Pro Arg Arg Gla Val Cys Gla/Glu Leu Asn Pro Asp Cys Asp Glu Leu Ala Asp His Ile Gly Phe Gln Glu Ala Tyr Arg Arg Phe Tyr Gly Pro Val (Sequence ID No. 3 or 4).

The anti-Glu¹⁷-osteocalcin antibody according to the present invention may be a polyclonal antibody or monoclonal antibody. It may be a fragment of the antibody insofar as it has reactivity of the antibody.

The fragment of the antibody may be an F(ab')₂, Fab' or Fab fraction from which an Fc' or Fc region has been removed or a polymer thereof; or a chimeric antibody thereof.

The antibody according to the present invention can be prepared by using as an antigen peptide (or epitope) a peptide fragment indicated by Sequence ID No. 5 or fragment thereof containing a Glu residue.

Here, the term "peptide fragment (Val Pro Tyr Pro Asp Pro Leu Glu Pro Arg Arg Gla Val) indicated by Sequence ID No. 5" as used herein means a polypeptide which is a peptide fragment of an osteocalcin from position 10 to position 22 and is thus composed of 13 amino acids with the Gla residue at position 17 having been substituted by a Glu residue.

The term "fragment thereof containing a Glu residue" as used herein means a portion of the fragment containing a Glu residue in the peptide fragment indicated by Sequence ID No. 5.

The term "epitope" means an antigen determinant having an already-known structure and in the case of a protein (polypeptide), it is generally formed of at least 6 amino acids (it is disclosed in JP-A-60-500684 that a polypeptide composed of six amino acids binds to an antibody). Upon preparation of an anti-Glu¹⁷-osteocalcin antibody according to the present invention, not only a peptide fragment indicated by Sequence ID No. 5 but also a sub-fragment thereof containing a Glu residue can be used as an epitope. Preferably, a peptide fragment having at least 6 continuous amino acids is selected.

In other words, the anti-Glu¹⁷-osteocalcin antibody of the present invention can be prepared using as an epitope a peptide fragment continuously composed of at least six amino acids.

Among the antibodies so prepared, particularly preferred is an antibody which does not bind to a peptide fragment indicated by Sequence ID No. 3 or 4 (Tyr Leu Tyr Gln Trp Leu Gly Ala Pro Val Pro Tyr Pro Asp Pro Leu Gla Pro Arg Arg Gla Val Cys Gla/Glu Leu Asn Pro Asp Cys Asp Glu Leu Ala Asp His Ile Gly Phe Gln Glu Ala Tyr Arg Arg Phe Tyr Gly Pro Val) and/or a peptide fragment indicated by Sequence ID No. 6 (Val Pro Tyr Pro Asp Pro Leu Gla Pro Arg Arg Gla Val).

The antibody according to the present invention can be used as a reagent for detecting low carboxylated osteocalcin, particularly, a Glu¹⁷-osteocalcin and it is also useful for judging the risk of the fracture caused by osteofragility or osteoporosis, which has recently been a problem.

The reagent of the present invention for detecting Glu¹⁷-osteocalcin can be prepared by the incorporation of the antibody of the present invention. By blending, for example, a standard antigen, a solution for diluting the antigen, a color developing agent, a substrate dissolving solution, a washing liquid, a reaction terminating liquid and ordinarily employed components used as raw materials for a detection reagent, a target kit for detecting Glu¹⁷-osteocalcin can be prepared.

Glu¹⁷-osteocalcin in a sample such as blood, plasma, serum, urea or the like can be assayed by labeling the Glu¹⁷-osteocalcin with a fluorescent or light-emitting substance, an enzyme such as peroxidase or a radioisotope such as ¹²⁵I and then reacting the resulting labeled Glu¹⁷-osteocalcin competitively with an antigen in the sample and an antibody of the present invention.

The antibody of the present invention has properties as described above. Among them, examples of the particularly preferred antibody include 1) an antibody which specifically binds to Glu¹⁷-osteocalcin; 2) an antibody which specifically binds to Glu¹⁷- and Gla²¹-osteocalcins; 3) an antibody which specifically binds to a peptide fragment indicated by Sequence ID No. 1 or 2 and does not bind to a peptide fragment indicated by Sequence ID No. 3 or 4; 4) an antibody which specifically binds to a peptide fragment indicated by Sequence ID No. 5 and does not bind to a peptide fragment indicated by Sequence ID No. 6; and 5) which specifically binds to a peptide fragment indicated by [Sequence ID No. 1 or 2] and [Sequence ID No. 5] and does not bind to a peptide fragment indicated by [Sequence ID No. 3 or 4] and [Sequence ID No. 6].

Accordingly, the use of the antibody having such properties makes it possible to discriminate four variant types of low carboxylated osteocalcins having a Glu residue at position 17 and moreover, out of them, discriminate two variant types (Glu residue at position 17, Gla residue at position 21, Gla residue or Glu residue at position 24) of low-carboxylated osteocalcins having a Glur residue at position 17 and a Gla residue at position 21.

### Modes for Carrying Out the Invention

The antibody of the present invention can be formed as follows: The peptide fragment of Glu¹⁷-osteocalcin indicated by Sequence ID No. 5 is used as an antigen, or a complex of it with a carrier protein (BSA [bovine serum albumin], OVA [ovalbumin] or the like) is formed as needed. The resulting antigen or the complex is inoculated to an animal, whereby the animal is immunized with the antigen. It is also possible to immunize by using a MAP system (Multiple antigen peptide system) developed by Tam JP [Tam JP, "Synthetic Peptide: Approaches to Biological Problems", 3-18(1989); Tam JP, PNAS USA, 85, 5409(1988); Tam et al., J. Exp Med., 171, 299(1990); Tam and Lu., PNAS USA, 86, 9084(1989)]. Then, the antigen forming cells obtained from the spleen or lymph node of the immunized animal are fused with myeloma cells and a hybridoma which forms an antibody exhibiting strong specificity to the peptide fragment indicated by Sequence ID No. 5 and the low carboxylated osteocalcin (Glu¹⁷-osteocalcin) is selected, whereby the antibody is prepared. The operation is carried out in a manner known to date.

Usable as an immunizing antigen is any one of a fragment of natural Glu¹⁷-osteocalcin or Glu¹⁷-osteocalcin induced by warfarin, a fragment of Glu¹⁷-osteocalcin obtained by genetic recombination technique, a fragment of natural osteocalcin or osteocalcin, which has been prepared by the gene recombination, after thermal or chemical decarboxylation, and the fragment produced by a chemically synthesizing technique.

When natural Glu¹⁷-osteocalcin is used as an immunizing antigen, it is not practical to separate and purify it because its concentration in a biological sample is very low (its content in the blood is on the order of pg/ml so that several tons of blood are necessary for securing a sufficient amount to be supplied for immunization). If the sample can be used in a large amount, it is possible to carry out separation and purification by using known methods in combination. Examples of the known method include salting out, solvent precipitation, dialysis gel filtration, electrophoresis, ion exchange chromatography, affinity chromatography and reversed-phase high-performance liquid chromatography.

Accordingly, it is practical to prepare a fragment peptide of Glu¹⁷-osteocalcin for use as an immunizing antigen by employing a method such as chemical synthesis, gene recombination or decomposition of a natural substance. More conveniently, it can be prepared by the manner known to date by using a peptide synthesizer.

For the preparation of a complex of an antigen and a carrier protein, various condensation agents can be used, with glutaraldehyde, carbodiimide, maleimide active ester and the like being preferably used.

As a carrier protein, conventionally-used ones such as bovine serum albumin, thyroglobulin and hemocyanin can be used. It is generally coupled with the antigen in an amount of 1 to 5 times as much as that of the antigen.

Examples of the animal to be immunized include mouse, rat, rabbit and guinea pig. It is inoculated by subcutaneous, intramuscular or intraperitoneal administration. Upon administration, the antigen or the complex may be mixed with a Freund's complete adjuvant (FCA) or Freund's incomplete adjuvant (FIA). The administration is generally effected once in two to 5 weeks. A polyclonal antibody is obtained by collecting the blood from the immunized animal, separating the serum and purifying the antibody from the serum by the conventionally-employed method such as ammonium sulfate salting out, ion exchange chromatography or affinity chromatography. The antibody forming cells obtained from the spleen or lymph node of the immunized animal is cell-fused with myeloma cells, followed by isolation as a hybridoma. Examples of the myeloma cells used here include those derived from mouse, rat and human being. The myeloma cells are preferred to have the same origin with that of the antibody forming cells but those from another species can sometimes be used.

The cell fusion can be conducted following a known method, for example, that proposed by Kehler and Milstein [Nature, 256, 495(1975)]. As a fusion accelerator, polyethylene glycol and Sendai virus can be mentioned as examples. The cell fusion can be carried out by reacting the antibody forming cells with myeloma cells generally in the presence of polyethylene glycol (average molecular weight: 1,000 to 4,000) having a concentration of 20 to 50%, at 20 to 40°C, preferably 30 to 37°C, generally at a vial count ratio of the former to the latter being about 1:1 to 10:1, for about 1 to 10 minutes. To the screening of the hybridoma for the formation of an anti-Glu¹⁷-osteocalcin antibody, various immunochemical methods can be applied. Examples include ELISA (enzyme-linked immunosorbent assay) method using a microplate coated with Glu¹⁷-osteocalcin, EIA (enzyme immunoassay) method using a microplate coated with an anti-immunoglobulin antibody, and Western blot analysis in which a nitrocellulose transcription membrane is used subsequent to the electrophoresis of a sample containing Glu¹⁷-osteocalcin.

The hybridoma is subjected further to cloning from the well, for example, by limiting dilution analysis. Selective culture of the hybridoma is conducted generally on a medium for animal cells, which medium includes 10 to 20% bovine fetal serum (ex. RPMI 1640) and has been added with HAT (hypoxanthin, aminopterin and thymidine). The clone so obtained is transplanted to the abdominal cavity of a BALB/c mouse, to which Bristan has been administered in advance, and 10 to 14 days later, an ascitic fluid containing a monoclonal antibody in a high concentration is collected, which can be used as a raw material for the purification of an antibody. Alternatively, the clone obtained above is cultured and the resulting cultured substance can be used as a raw material for the purification of an antibody. The monoclonal antibody may be recovered by the method conventionally known as a purification method of immunoglobulin. The recovery can easily be accomplished by utilizing sulfate ammonium fractionation, PEG fractionation, ethanol fractionation, anion exchanger or affinity chromatography.

The immunological assay using an anti-Glu¹⁷-osteocalcin antibody obtained by the present invention makes it possible to carry out qualitative and quantitative analysis of a Glu¹⁷-osteocalcin (including carboxylated osteocalcin such as a normal osteocalcin which comes to have a Glu residue at position 17 owing to the decarboxylation of a Gla residue at the same position and an osteocalcin having PIVKA (protein induced by vitamin K absence or antagonist) at position 17 which has appeared owing to the metabolic disorder of vitamin K or the like) in a biological sample.

When used in the immunological assay, a biological sample such as blood, plasma, serum or urea can be properly treated as needed. Those subjected to cell separation or extraction can also be used as samples. To these samples, conventionally known assays such as immunological tissue staining method, immunoassay such as ELISA and RIA (radioimmunoassay), agglutination, competitive assay or sandwich assay can be applied. The immunological tissue staining method and immunoassay are effected either by a direct method using a labeling antibody or an indirect method using a labeled antibody. Examples of the labeling agent include conventionally known labeling substances such as fluorescent or light-emitting substance, radioactive substance, enzyme, metal and pigment.

### Brief Description of the drawings:

FIG. 1 illustrates the dependence, on a calcium concentration in a buffer, of a standard curve of Glu¹⁷-osteocalcin in the radioimmunoassay using an anti-Glu¹⁷-osteocalcin antibody. As a standard substance, Glu¹⁷-osteocalcin (1-49) "○" (circle) or Gla¹⁷-osteocalcin (1-49) "◇" (square) was used.
FIG. 2 illustrates a change by age in the Glu¹⁷-osteocalcin concentration in plasma in 322 cases of normal women.
FIG. 3 illustrates a correlation between the concentration of Glu¹⁷-osteocalcin in plasma and a lumbar bone mineral quantity, Z score, in 322 cases of normal women.
FIG. 4 illustrates a correlation between a Glu¹⁷-osteocalcin concentration in plasma and the absolute value of a lumbar bone mineral quantity in each age group of women.

### Examples

The present invention will hereinafter be described in detail specifically by examples, but it should however borne in mind that the present invention will not be limited to or by the following examples.

### Example 1. Synthesis of antigen peptide

The peptide indicated by Sequence ID No. 5 which was to be used as an antigen was synthesized by an automatic peptide synthesizer manufactured by Applied Biosystems/U.S.A. First, a protective peptide resin corresponding to it was synthesized successively from the C terminal in accordance with a solid-phase synthesizing method using a high-molecular resin carrier. The protected peptide resin was then subjected to acid treatment and the target crude peptide was purified by reversed-phase high-performance liquid chromatography, whereby a target peptide for antigen was obtained.

The resulting peptide was composed of 13 residues, that is, Val Pro Tyr Pro Asp Pro Leu Glu Pro Arg Arg Gla Val and it was the peptide fragment from position 10 to position 22 of the normal osteocalcin except that the Gla residue at position 17 was substituted with a Glu residue. A cysteine residue was added to the N terminal for conjugation.

### Example 2. Preparation of antibody

The peptide so synthesized bound to BSA, OVA or the like to have high molecules and it was inoculated to the mouse as an immunogen. Described specifically, after sampling the blood for measuring a titer of un-immunized serum on Day 0, an emulsion of the above-described peptide with FCA was prepared. To the abdominal cavities of two mice (BALB/C), the resulting emulsion was administered at a dose of 50 µg/mouse periodically for 2 to 3 months, whereby the mouse were immunized.

The spleen cells were then collected from the mice and fused with Myeloma cells P3U1. The fused cells were cultured and selected on a HAT medium. The fused cells which reacted with the peptide fragment indicated by Sequence ID No. 5 and low-carboxylated osteocalcin (Glu¹⁷-osteocalcin) indicated by Sequence ID No. 1 or 2; and did not react with any one of the osteocalcin indicated by Sequence ID No. 3 or 4 and the peptide fragment indicated by Sequence ID No. 6 were cloned. Only the cells secreting IgG were selected and IgG in the culture supernatant was purified by protein A or protein G, whereby a purified monoclonal antibody was obtained.

### Example 3. Detection of titer, etc.

The properties and a titer of the antibody were studied by the ELISA method.

First, the antigen peptide was coated on a microplate (H type, A type; produced by Sumitomo Bakelite). After blocking the plate with a casein solution, the culture supernatant or purified monoclonal antibody was properly diluted in steps and added, whereby an antigen antibody reaction was effected on a solid phase to form an antigen-antibody complex. To the resulting complex, an enzyme-labeled second antibody (anti mouse IgG antibody) was reacted. By measuring the activity of the enzyme bound onto the solid phase after washing, presence or absence of the formation of the antibody, change in a titer and specificity were studied.

The antibody obtained above reacted with the peptide indicated by Sequence ID No. 5 but did not show a cross reaction with the peptide indicated by Sequence ID No. 6.

### Example 4. Radioimmunoassay of Glu¹⁷-osteocalcin

Glu¹⁷-osteocalcin was labeled with ¹²⁵I by the enzyme method. Then, radioimmunoassay of Glu¹⁷-osteocalcin was effected using an anti-Glu¹⁷-osteocalcin antibody, Glu¹⁷-osteocalcin (1-49) or Gla¹⁷-osteocalcin (1-49) as a standard substance, 0.5% bovine serum albumin and a 5 mM tris-hydrochloric acid buffer (pH 7.4) as a buffer.

Moreover, in order to study the dependence on the calcium concentration in the buffer in each radioimmunoassay, calcium chloride was added to give a concentration of 0.03 to 10 mM. When the calcium concentration in the buffer was 0 mM, 1 mM of EGTA was added. The B/F separation was conducted by the double antibody assay.

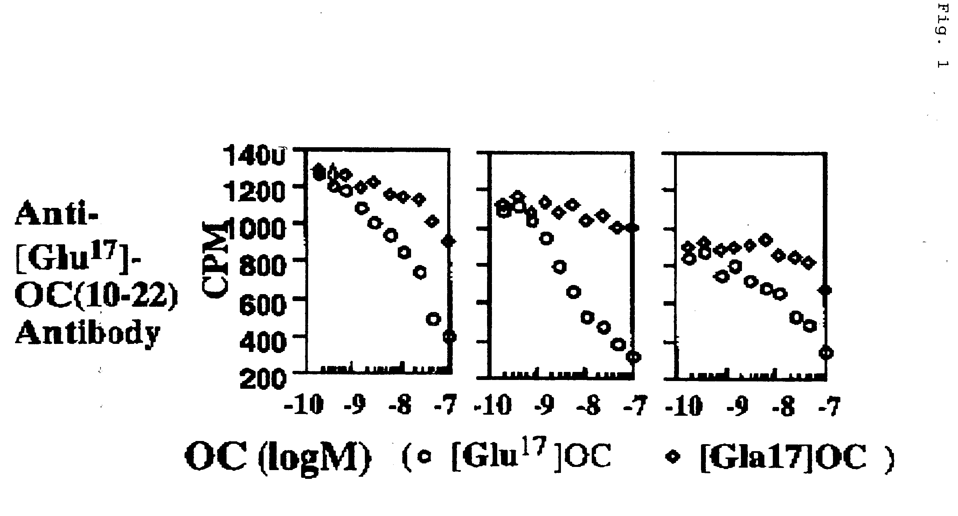

FIG. 1 illustrates the results of the study concerning the dependence, on a calcium concentration in a buffer, of the standard curves of Glu¹⁷-osteocalcin (1-49) and Gla¹⁷-osteocalcin in the radioimmunoassay.

In each radioimmunoassay, two standard curves exhibited the dependence on the calcium concentration in the buffer. In the radioimmunoassay conducted using as an antibody an anti-Glu¹⁷-osteocalcin antibody obtained in Example 2, the standard substance Glu¹⁷-osteocalcin (1-49) formed a good standard curve when the calcium concentration in the buffer was 0 mM or 0.03 mM. Moreover, the antibody did not recognize Gla¹⁷-osteocalcin (1-49) until the concentration became high so that it was possible to separate and discriminate Glu¹⁷-osteocalcin (1-49) from Gla¹⁷-osteocalcin (1-49).

### Example 5. Measurement of Glu¹⁷-osteocalcin in human plasma

For the radioimmunoassay of the concentration of Glu¹⁷-osteocalcin in human plasma, an anti-Glu¹⁷-osteocalcin antibody was used and also 0.5% bovine serum albumin, 1 mM EGTA and 5 mM Tris-hydrochloric acid solution (pH 7.4) were employed as a measuring buffer.

Any dilution curve of the plasma exhibited a good parallel relationship with the standard curve of Glu¹⁷-osteocalcin (1-49).

As can be seen from the above results, there also exists Glu¹⁷-osteocalcin immmunoactivity in human blood and this radioimmunoassay is useful for the measurement of Glu¹⁷-osteocalcin in the human body.

In the following examples, upon measuring the concentration of Glu¹⁷-osteocalcin in the human plasma, a dilution curve of plasma was prepared for each serum specimen. Values ± 20% end portions of the standard curve were eliminated and only the values of the diluted plasma specimen positioned within central 80% of the standard curve were adopted.

### Example 6. Change by age of Glu¹⁷-osteocalcin concentration in plasma in women

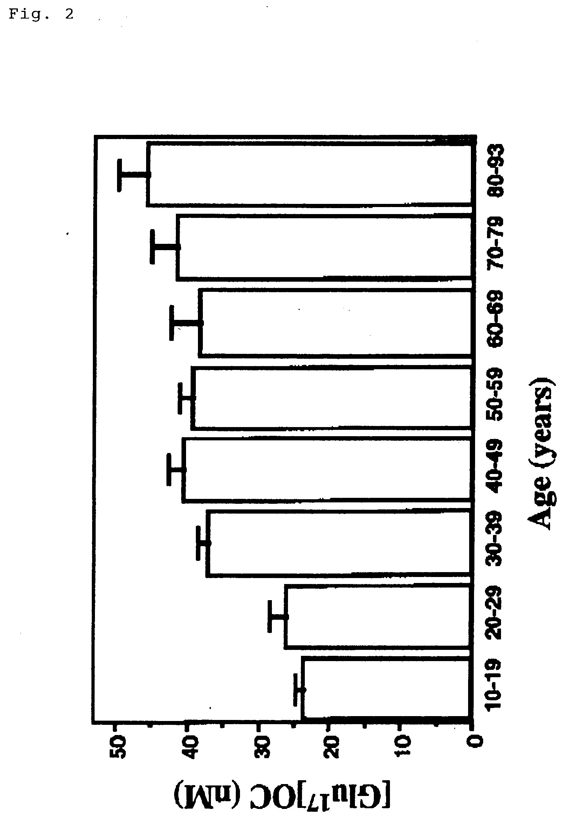

FIG. 2 illustrates a change of an average value of a Glu¹⁷-osteocalcin concentration with aging in plasma in 322 cases of normal women.

From 16 to 93-year-old 322 normal women who showed a body mass index (BMI) within a normal range (18 to 26) and could walk by themselves, blood was collected through an EGTA tube in morning hunger. Immediately after the collection, plasma was separated from the blood, stored at -20°C and then provided for use in the measurement.

From the results, it has been understood that the concentration of Glu¹⁷-osteocalcin in plasma increased with age, and that an increasing rate was particularly high in the case of women near or just after menopause and also the women of advanced age. The above increase in the Glu¹⁷-osteocalcin concentration in plasma with aging is similar to the report by Plantalech and et al. [Plantalech L, Guillaumont M, Vergnaud P, Leclercq M, Delmas PD., "Impairment of gamma carboxylation of circulating osteocalcin (bone Gla protein) in elderly women", J. Bone Miner. Res., **6**, 1211-16(1991)].

### Example 7. Correlation between Glu¹⁷-osteocalcin concentration in plasma and lumbar bone mineral quantity in normal women

A correlation, in women, between a Glu¹⁷-osteocalcin concentration in plasma and a bone mineral quantity of the second to fourth lumbar as measured by a double X-ray absorbed-bone mineral density measuring apparatus (DPX manufactured by Lunar Inc.) was studied.

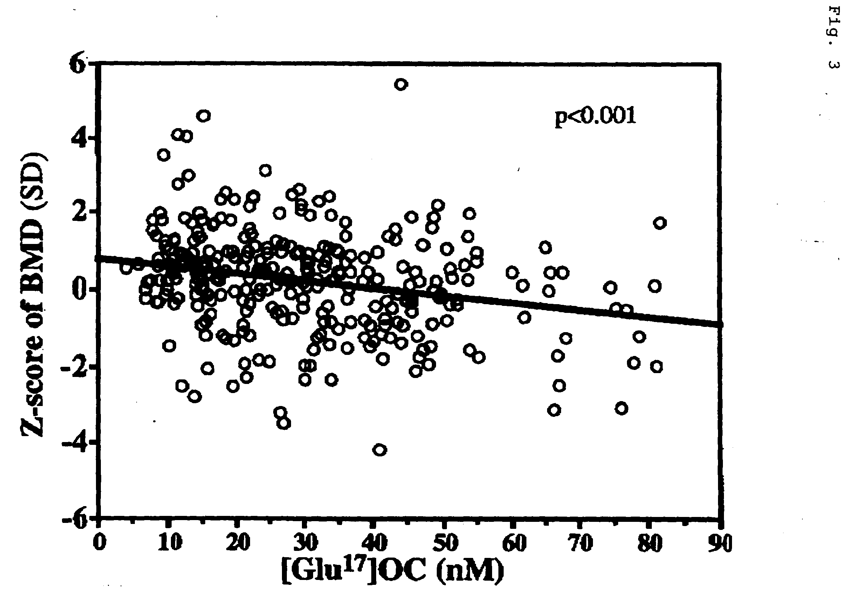

FIG. 3 illustrates a correlation, in 322 female cases, between the Glu¹⁷-osteocalcin concentration in plasma and a Z score which is a value indicating how much the bone mineral amount of each person is different from the Japanese average of each age supposing that the Japanese average of the lumbar bone mineral quantity of each age is 0 and the standard deviation is 1. It has been found that, in all the cases, when the concentration of Glu¹⁷-osteocalcin in plasma is higher, the lumbar bone mineral quantity, Z score, lowers and there is a negative correlation of p<0.001 between them.

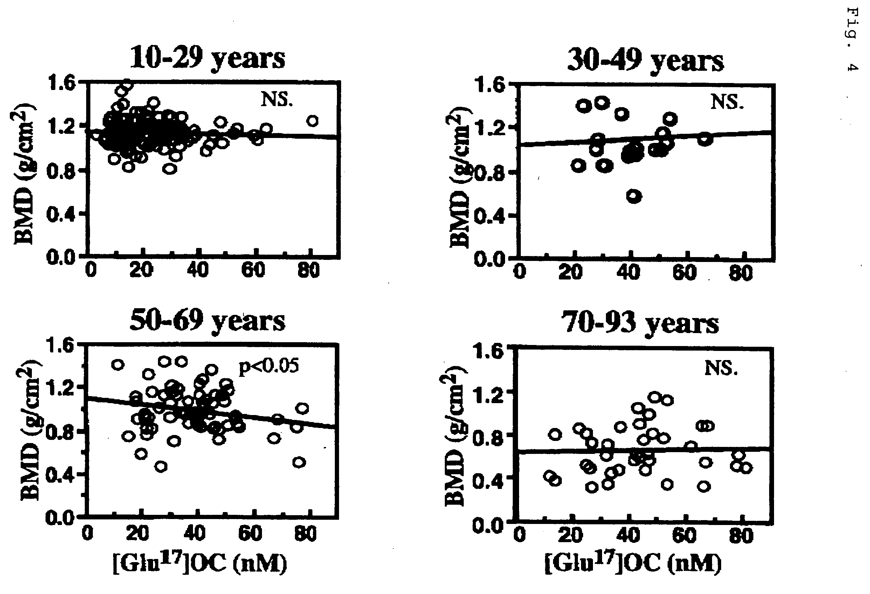

FIG. 4 illustrates the correlation between a Glu¹⁷-osteocalcin concentration in plasma and an absolute value of a lumbar bone mineral quantity, classified by four women groups, that is, a 16 to 29 year-old younger group, a 30 to 49 year-old meridian group, a 50 to 69 year-old postmenopausal group and 70 to 93 year-old advanced age group. It has been found that in the 50 to 69 year-old postmenopausal group among these groups, there is a negative correlation between a Glu¹⁷-osteocalcin concentration in plasma and the absolute value of a lumbar bone mineral content.

As described above, it has been found that there is a significant negative correlation of p<0.001, in normal women, between a Glu¹⁷-osteocalcin concentration in plasma and the absolute value of a lumbar bone mineral quantity and, irrespective of age, the Glu¹⁷-osteocalcin concentration in plasma exhibited a high value in the case where the bone mineral quantity was lower than that suited to her age. It has also been observed that in the study of a correlation between the Glu¹⁷-osteocalcin concentration in plasma and the absolute value of a lumbar bone mineral quantity classified by four women group, there was a negative relationship between a Glu¹⁷-osteocalcin concentration in plasma and an absolute value of a lumbar bone mineral content in the 50 to 69 year-old postmenopausal group. Accordingly, it can be presumed that the high concentration of Glu¹⁷-osteocalcin in plasma takes part in a marked decrease in bone mineral density of women of the above age, that is, postmenopausal osteoporosis. The above study was carried out with the normal women as its object, but the assay system of the present invention is also useful for elucidating the pathophysiological significance of low carboxylated osteocalcin in various bone diseases and also the action of vitamin K, as well as the above-described osteoporosis which occurs with high frequency.

As described above, the use of the antibody of the present invention makes it possible to detect and assay Glu¹⁷-osteocalcin easily and is therefore very useful for the diagnosis of bone diseases relating to Glu¹⁷-osteocalcin.

SEQUENCE DESCRIPTION

SEQ ID NO: 1
SEQUENCE LENGTH: 49
SEQUENCE TYPE: amino acid
TOPOLOGY: linear
MOLECULE TYPE: protein
ORIGINAL SOURCE
ORGANISM: human
SEQUENCE DESCRIPTION
Tyr Leu Tyr Gln Trp Leu Gly Ala Pro Val Pro Tyr Pro Asp Pro Leu
1               5                   10                  15
Glu Pro Arg Arg Xaa Val Cys Glu Leu Asn Pro Asp Cys Asp Glu Leu
            20                  25                  30
Ala Asp His Ile Gly Phe Gln Glu Ala Tyr Arg Arg Phe Tyr Gly Pro
        35                  40                  45
Val

SEQ ID NO: 2
SEQUENCE LENGTH: 49
SEQUENCE TYPE: amino acid
TOPOLOGY: linear
MOLECULE TYPE: protein

ORIGINAL SOURCE
ORGANISM: human
SEQUENCE DESCRIPTION
Tyr Leu Tyr Gln Trp Leu Gly Ala Pro Val Pro Tyr Pro Asp Pro Leu
1               5                   10                  15
Glu Pro Arg Arg Xaa Val Cys Xaa Leu Asn Pro Asp Cys Asp Glu Leu
            20                  25                  30
Ala Asp His Ile Gly Phe Gln Glu Ala Tyr Arg Arg Phe Tyr Gly Pro
        35                  40                  45
Val

SEQ ID NO: 3
SEQUENCE LENGTH: 49
SEQUENCE TYPE: amino acid
TOPOLOGY: linear
MOLECULE TYPE: protein
ORIGINAL SOURCE
ORGANISM: human
SEQUENCE DESCRIPTION
Tyr Leu Tyr Gln Trp Leu Gly Ala Pro Val Pro Tyr Pro Asp Pro Leu
1               5                   10                  15
Xaa Pro Arg Arg Xaa Val Cys Glu Leu Asn Pro Asp Cys Asp Glu Leu
            20                  25                  30
Ala Asp His Ile Gly Phe Gln Glu Ala Tyr Arg Arg Phe Tyr Gly Pro
        35                  40                  45

Val

SEQ ID NO: 4
SEQUENCE LENGTH: 49
SEQUENCE TYPE: amino acid
TOPOLOGY: linear
MOLECULE TYPE: protein
ORIGINAL SOURCE
ORGANISM: human
SEQUENCE DESCRIPTION
Tyr Leu Tyr Gln Trp Leu Gly Ala Pro Val Pro Tyr Pro Asp Pro Leu
1               5                   10                  15
Xaa Pro Arg Arg Xaa Val Cys Xaa Leu Asn Pro Asp Cys Asp Glu Leu
            20                  25                  30
Ala Asp His Ile Gly Phe Gln Glu Ala Tyr Arg Arg Phe Tyr Gly Pro
        35                  40                  45
Val

SEQ ID NO: 5
SEQUENCE LENGTH: 13
SEQUENCE TYPE: amino acid
TOPOLOGY: linear
MOLECULE TYPE: protein
ORIGINAL SOURCE
ORGANISM: human

SEQUENCE DESCRIPTION
Val Pro Tyr Pro Asp Pro Leu Glu Pro Arg Arg Xaa Val
1               5                   10

SEQ ID NO: 6
SEQUENCE LENGTH: 13
SEQUENCE TYPE: amino acid
TOPOLOGY: linear
MOLECULE TYPE: protein
ORIGINAL SOURCE
ORGANISM: human
SEQUENCE DESCRIPTION
Val Pro Tyr Pro Asp Pro Leu Xaa Pro Arg Arg Xaa Val
1               5                   10

## Claims

1. An anti-Glu¹⁷-osteocalcin antibody or fragment thereof which specifically binds to Glu¹⁷-osteocalcin which is an osteocalcin having a Glu residue at position 17 thereof or to an osteocalcin fragment containing the 17th Glu residue.

2. The anti-Glu¹⁷-osteocalcin antibody or fragment thereof according to claim 1, which specifically binds to Gla²¹-osteocalcin which is an osteocalcin having a Gla residue at position 21 or to an osteocalcin fragment containing the 21st Gla residue.

3. The anti-Glu¹⁷-osteocalcin antibody or fragment thereof according to claim 1 or 2, which specifically binds to a peptide fragment indicated by Sequence ID No. 1 or 2 wherein Xaa represents a Gla residue.

4. The anti-Glu¹⁷-osteocalcin antibody or fragment thereof according to any one of claims 1 to 3, which binds to neither a peptide fragment indicated by Sequence ID No. 3 nor 4 wherein Xaa represents a Gla residue.

5. The anti-Glu¹⁷-osteocalcin antibody or fragment thereof according to claim 1 to 4, which specifically binds to a peptide fragment indicated by Sequence ID No. 5 wherein Xaa represents a Gla residue or fragment thereof containing a Glu residue.

6. The anti-Glu¹⁷-osteocalcin antibody or fragment thereof according to any one of claims 1 to 5, which binds to a peptide indicated by Sequence ID No. 5 wherein Xaa represents a Gla residue or fragment thereof containing a Glu residue and does not bind to a peptide fragment indicated by Sequence ID No. 6 wherein Xaa represents a Gla residue.

7. The anti-Glu¹⁷-osteocalcin antibody or fragment thereof according to any one of claims 1 to 6, which has been obtained using as an antigen a peptide fragment indicated by Sequence ID No. 5 wherein Xaa represents a Gla residue or fragment thereof containing a Glu residue.

8. The anti-Glu¹⁷-osteocalcin antibody or fragment thereof according to any one of claims 1 to 7, wherein the antibody is a monoclonal antibody.

9. A reagent for detecting Glu¹⁷-osteocalcin, which comprises an anti-Glu¹⁷-osteocalcin antibody or fragment thereof as claimed 1 to 8.

10. An immunoassay of a Glu¹⁷-osteocalcin in a sample, which comprises labeling Glu¹⁷-osteocalcin with a fluorescent or light-emitting substance, an enzyme or a radioisotope and detecting the Glu¹⁷-osteocalcin in accordance with the competitive assay by using an anti-Glu¹⁷-osteocalcin antibody or fragment thereof as claimed in claim 1 to 8.

11. The anti-Glu¹⁷-osteocalcin antibody or fragment thereof according to any one of claims 1 to 7, wherein the fragment thereof containing a Glu residue is formed of at least six continuous peptide pieces.
